(19) Europäisches Patentamt / European Patent Office / Office européen des brevets

(11) **EP 3 476 273 A1**

(12) **EUROPEAN PATENT APPLICATION**
published in accordance with Art. 153(4) EPC

(43) Date of publication:
**01.05.2019 Bulletin 2019/18**

(51) Int Cl.:
**A61B 1/06** (2006.01)    **A61B 1/045** (2006.01)
**G02B 23/26** (2006.01)

(21) Application number: **17819630.9**

(22) Date of filing: **26.04.2017**

(86) International application number:
**PCT/JP2017/016461**

(87) International publication number:
**WO 2018/003263 (04.01.2018 Gazette 2018/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.06.2016 JP 2016126419**
**22.03.2017 JP 2017055339**

(71) Applicant: **Sony Corporation**
**Tokyo 108-0075 (JP)**

(72) Inventors:
• **MURAMATSU, Hirotaka**
  **Tokyo 108-0075 (JP)**
• **YAMAGUCHI, Takashi**
  **Tokyo 108-0075 (JP)**

(74) Representative: **D Young & Co LLP**
**120 Holborn**
**London EC1N 2DY (GB)**

(54) **OBSERVATION DEVICE AND CONTROL METHOD FOR OBSERVATION DEVICE**

(57)    [Object] To provide an observation apparatus capable of capturing an observation image having appropriate color discriminability regardless of color of an observation target and a method of controlling the observation apparatus.

[Solution] The observation apparatus includes: a plurality of light sources configured to emit light different in wavelength spectrum; an optical system configured to emit observation light obtained by combining respective beams of light emitted from the plurality of light sources to an observation target; an image generation unit configured to generate an observation image on the basis of light from the observation target; a light quantity ratio calculation processing unit configured to determine a light quantity ratio of each of the plurality of light sources on the basis of information related to a color of the generated observation image; and a controller configured to control the plurality of light sources on the basis of the determined light quantity ratio.

FIG. 4

**Description**

Technical Field

The present disclosure relates to an observation apparatus and a method of controlling the observation apparatus.

Background Art

For a recent observation apparatus for observing a surgical site of a patient, such as endoscopic instruments and microscopic instruments, it becomes common to use light from a plurality of light sources for illumination.

The use of a white light source in conjunction with a laser light source having a narrow wavelength band, in one example, as a light source of the observation apparatus for illumination is considered. Such an observation apparatus combines the laser light source having the narrow wavelength band with optical absorption property of a particular tissue such as a blood vessel, so it is possible to observe the particular tissue with emphasis.

In one example, Patent Literatures 1 and 2 below disclose endoscopic instruments that include a semiconductor light-emitting device and use light emitted from a first light source and a second light source having mutually different light emission wavelengths as observation light.

Citation List

Patent Literature

    Patent Literature 1: JP 2011-010998A
    Patent Literature 2: JP 2015-091351A

Disclosure of Invention

Technical Problem

In the endoscopic instrument disclosed in the above-mentioned Patent Literature 1 or 2, however, light emitted from the first light source and light emitted from the second light source are combined at a preset light quantity ratio or a user-specified light quantity ratio and then used as observation light. Thus, in the endoscopic instrument disclosed in the above-mentioned Patent Literatures 1 or 2, there is a possibility that color discriminability of an observation image is inappropriate depending on the combination of the wavelength spectrum of the observation light and the color of an observation target.

In view of this, the present disclosure provides a novel and improved observation apparatus capable of capturing an observation image having appropriate color discriminability regardless of color of an observation target and method of controlling the observation apparatus.

Solution to Problem

According to the present disclosure, there is provided an observation apparatus including: a plurality of light sources configured to emit light different in wavelength spectrum; an optical system configured to emit observation light obtained by combining respective beams of light emitted from the plurality of light sources to an observation target; an image generation unit configured to generate an observation image on the basis of light from the observation target; a light quantity ratio calculation processing unit configured to determine a light quantity ratio of each of the plurality of light sources on the basis of information related to a color of the generated observation image; and a controller configured to control the plurality of light sources on the basis of the determined light quantity ratio.

In addition, according to the present disclosure, there is provided a method of controlling an observation apparatus, the method including: emitting light different from each other in wavelength spectrum from a plurality of light sources; emitting observation light obtained by combining respective beams of emitted light to an observation target; generating an observation image on the basis of light from the observation target; determining, by a calculation processing device, a light quantity ratio of each of the plurality of light sources on the basis of information related to a color of the generated observation image; and controlling the plurality of light sources on the basis of the determined light quantity ratio.

According to the present disclosure, it is possible to control a light quantity ratio of a plurality of light sources that emit light beams different from each other in wavelength spectrum on the basis of information related to the color of the observation image to obtain satisfactory color discriminability, thereby generating observation light obtained by combining light emitted from the plurality of light sources.

Advantageous Effects of Invention

According to the present disclosure as described above, it is possible to capture an observation image having appropriate color discriminability regardless of the color of the observation target.

Note that the effects described above are not necessarily limitative. With or in the place of the above effects, there may be achieved any one of the effects described in this specification or other effects that may be grasped from this specification.

Brief Description of Drawings

FIG. 1 is a schematic diagram illustrating a general configuration of an observation apparatus according to an embodiment of the present disclosure.

FIG. 2 is a graphic diagram illustrating comparison between wavelength spectra of light emitted from various light sources.

FIG. 3 is a schematic diagram illustrated to describe an optical system of a light source unit included in an observation apparatus according to a first embodiment of the present disclosure.

FIG. 4 is a block diagram illustrating a configuration of the observation apparatus according to the present embodiment.

FIG. 5 is an example of an observation image in which a noticed area is set through an input device.

FIG. 6 is a flowchart illustrated to describe an example of a method of controlling the observation apparatus according to the present embodiment.

FIG. 7 is a block diagram illustrating a configuration of an information processing device included in an observation apparatus according to a second embodiment of the present disclosure.

FIG. 8 is a flowchart illustrated to describe an example of a method of controlling the observation apparatus according to the present embodiment.

FIG. 9 is a block diagram illustrating a configuration of an information processing device included in an observation apparatus according to a third embodiment of the present disclosure.

FIG. 10 is a flowchart illustrated to describe an example of a method of controlling the observation apparatus according to the present embodiment.

FIG. 11 is a diagram illustrated to describe another example of the method of controlling the observation apparatus according to the present embodiment.

FIG. 12 is a block diagram illustrating a configuration of an observation apparatus according to a modification of the present disclosure.

Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

Moreover, the description will be given in the following order.

1. Overview of technology according to present disclosure
2. First embodiment

2.1. Configuration of light source
2.2. Configuration of observation apparatus
2.3. Method of controlling observation apparatus

3. Second embodiment

3.1. Configuration of observation apparatus

3.2. Method of controlling observation apparatus

4. Third embodiment

4.1. Configuration of observation apparatus
4.2. Method of controlling observation apparatus

5. Modification
6. Concluding remarks

<1. Overview of technology according to present disclosure>

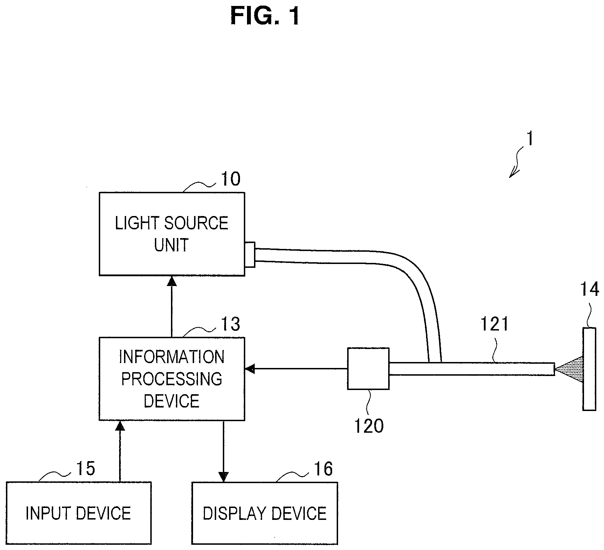

An overview of the technology according to the present disclosure is now described with reference to FIG. 1. FIG. 1 is a schematic diagram illustrating a general configuration of an observation apparatus according to an embodiment of the present disclosure.

An endoscopic instrument is now described taking as an example of the observation apparatus according to an embodiment of the present disclosure. However, the technology according to the present disclosure is not limited to an endoscopic instrument and is also applicable to a microscopic instrument. This will be described later with reference to <4. Modification>.

As illustrated in FIG. 1, the observation apparatus 1 includes a light source unit 10 that emits observation light to an observation target 14 via a lens barrel 121, an imaging unit 120 that photoelectrically converts light from the observation target 14, and an information processing device 13 that generates an observation image. In addition, the observation apparatus 1 can include a display device 16 that displays the generated observation image and an input device 15 that receives information input to the observation apparatus 1.

The light source unit 10 includes a plurality of light sources that emit light beams different from each other in wavelength spectrum, and combines light emitted from the plurality of light sources to generate observation light. The light source unit 10 is capable of generating observation light appropriate for various observation targets 14 by combining light having different wavelength spectra. In one example, the light source unit 10 can include a white light source that emits light in a wide wavelength band and a laser light source that emits light in a narrow wavelength band, or can include a plurality of light sources that emit light in the respective wavelength bands corresponding to colors such as red, green, and blue.

Moreover, in a case where the light source unit 10 uses a laser light source, the laser light source having high conversion efficiency from electrical power into light makes it possible for the power consumption of the observation apparatus 1 to be reduced. In addition, the light emitted from the laser light source has high optical coupling efficiency to a light guide (what is called light waveguide). Thus, the use of the laser light source in the light source unit 10 makes it possible to reduce light quantity loss in the optical system, thereby reducing the power consumption of the observation apparatus 1.

The lens barrel 121 includes therein a light guide extending to the distal end portion and guides the observation light emitted from the light source unit 10 to the observation target 14. In addition, the lens barrel 121 guides light reflected from the observation target 14 to the imaging unit 120. The lens barrel 121 can be formed in a rigid, substantially cylindrical shape or can be formed in a flexible, tubular shape.

The observation target 14 is, in one example, a biological tissue in a body cavity of a patient. The observation apparatus 1 inserts the lens barrel 121 into the body cavity of the patient to irradiate the observation target 14 with the observation light guided from the light source unit 10, and captures light reflected from the observation target 14 with the imaging unit 120 to acquire an image of the observation target 14.

The imaging unit 120 includes an image sensor capable of acquiring a color image, photoelectrically converts light from the observation target 14 into an electric signal by the image sensor, and outputs the converted electric signal to the information processing device 13. The image sensor included in the imaging unit 120 can be any of various well-known image sensors, such as charge-coupled device (CCD) image sensor or complementary metal-oxide-semiconductor (CMOS) image sensor.

The information processing device 13 generates the observation image obtained by capturing the observation target 14 by performing information processing on the electric signal that is input from the imaging unit 120. In addition, the information processing device 13 generates a control signal for the observation apparatus 1 on the basis of an input operation by the user through the input device 15. The information processing device 13 can be, in one example, a personal computer or the like equipped with central processing unit (CPU), read-only memory (ROM), random-access memory (RAM), or the like.

The display device 16 displays the observation image generated by the information processing device 13. The display device 16 can be, in one example, a cathode ray tube (CRT) display device, a liquid crystal display device, a plasma display device, organic electro luminescence (EL) display device, or the like. The user is able to operate the observation apparatus 1 to make a diagnosis of the observation target 14 or to perform medical treatment of the observation target 14 while visually recognizing the observation image displayed on the display device 16.

The input device 15 is an input interface and receives an input operation by the user. The input device 15 can be, in one example, an input device operated by the user, such as a mouse, a keyboard, a touch panel, a button, a switch, or a lever. The user is able to input various kinds of information or instructions to the observation apparatus 1 through the input device 15.

The inventors of the present disclosure have observed the observation targets 14 having different colors by irradiation with light from a plurality of light sources and so have found that color discriminability of the observation image varies depending on relationship between color of the observation target 14 and wavelength spectra of light emitted from the light source unit 10. In other words, the inventors of the present disclosure have found that the light sources having satisfactory color discriminability differ depending on the color of the observation target 14.

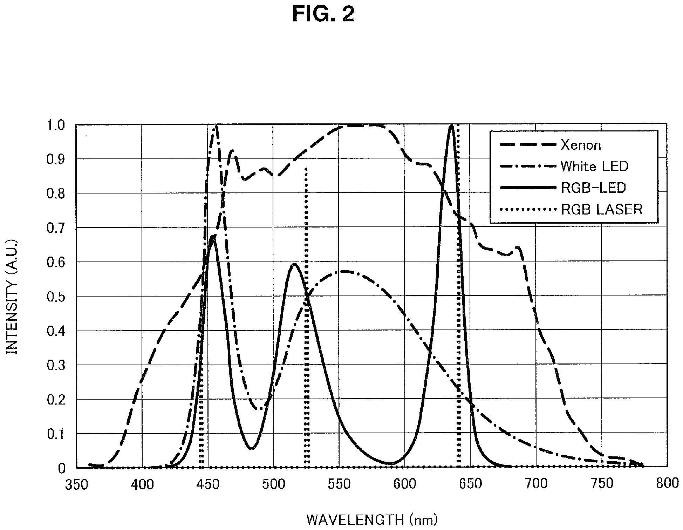

Specifically, as illustrated in FIG. 2, even if the light emitted from the light sources is the same white light, the wavelength spectrum differs depending on the type of the light sources. Moreover, FIG. 2 is a graphic diagram illustrating comparison between wavelength spectra of light emitted from various light sources.

Referring to FIG. 2, in one example, light emitted from a xenon lamp indicated by "Xenon" has a wide wavelength spectrum over the entire wavelength band of visible light. In addition, light emitted from a white light-emitting diode (LED) light source indicated by "White LED" has a wavelength spectrum having peaks around 450 nm and 550 nm. In addition, the observation light obtained by combining the light emitted from LEDs of the respective colors RGB (red, green, blue) indicated by "RGB-LED" has a wavelength spectrum having a narrow peak in the wavelength band corresponding to each color of RGB. Furthermore, the observation light obtained by combining the light emitted from the laser light sources of the respective colors RGB (red, green, blue) indicated by "RGB-laser" has three bright line spectra corresponding to the respective colors of RGB.

The light from these light sources was applied to a biological tissue sprayed with a pseudo sample exhibiting red color and a pseudo sample exhibiting yellow color and the color discriminability of the captured observation image was evaluated. The results are shown in Table 1 (for red color) and Table 2 (for yellow color). Moreover, the biological tissue sprayed with the pseudo sample exhibiting red color simulates the observation target 14 including blood or the like, and the biological tissue sprayed with the pseudo sample exhibiting yellow color simulates the observation target 14 including an adipose tissue or the like.

For comparison of color discriminability, a color difference between two colors $\Delta E$ at the point where the red pseudo sample or the yellow pseudo sample has buried depth of 0.3 mm and at the point where the buried depth is 0.4 mm was used. The color difference between two colors $\Delta E$ is a representation expressing a color difference between two colors as the distance in the L*a*b* space that is the human perceptual uniform space, and indicates that the greater the color difference between two colors $\Delta E$, the more different the color tint. The red or yellow color tone is stronger at the point where the buried depth of the color pseudo sample or yellow pseudo sample is 0.4 mm than the point where the buried depth is 0.3 mm. Thus, as the color difference between two colors $\Delta E$ is larger, it can be found that the color discriminability is higher by incorporating the difference in actual color tones.

[Table 1]

(Table 1: Biological tissue sprayed with red pseudo sample)

| Light source | Xenon lamp | White LED | RGB-LED | RGB LASER |
|---|---|---|---|---|
| $\Delta E$ | 1.19 | 1.01 | 1.59 | 1.76 |

[Table 2]

(Table 2: Biological tissue sprayed with yellow pseudo sample)

| Light source | Xenon lamp | White LED | RGB-LED | RGB LASER |
|---|---|---|---|---|
| $\Delta E$ | 3.05 | 3.53 | 2.32 | 2.07 |

Referring to Tables 1 and 2, it can be found that, in the pseudo sample exhibiting red color shown in Table 1, the color difference between two colors ΔE increases in the descending order of RGB laser, RGB-LED, xenon lamp, and white LED. On the other hand, in the pseudo sample exhibiting yellow color shown in Table 2, it is found that the color difference between two colors ΔE increases in the descending order of white LED, xenon lamp, RGB-LED, and RGB laser.

Thus, it can be found that the light source in which the color difference between two colors ΔE increases differs depending on the color of the observation object 14. The light sources used in the above description emit light whose wavelength spectrum is different, so it is assumed that the wavelength spectrum of appropriate observation light with satisfactory color discriminability differs depending on the color of the observation target 14.

Thus, in the observation apparatus in which the wavelength spectrum of the light emitted from the light source unit 10 is fixed, there was a possibility that the wavelength spectrum of the observation light is not appropriate depending on the color of the observation target 14, so the color discriminability of the observation image is deteriorated. In addition, even if the observation apparatus including a plurality of light sources that emit light different in wavelength spectrum allows the user to adjust a light quantity ratio of each light source, it is not practical for the user to adjust appropriately the light quantity ratio of each light source depending on variation in colors of the observation target 14. Thus, in such an observation apparatus, there was a possibility that the color discriminability of the observation image is deteriorated depending on the observation target 14.

The inventors of the present disclosure have conceived the technology according to the present disclosure on the basis of the above knowledge. The technology according to the present disclosure is the observation apparatus 1 that controls the light quantity ratio of each of a plurality of light sources included in the light source unit 10 on the basis of information related to a color of an observation image.

Specifically, the observation apparatus 1 can determine the light quantity ratio of each light source at which the color difference between two colors calculated from the observation image is maximized, and can control the plurality of light sources so that the determined light quantity ratio may be set. In addition, in the observation apparatus 1, the light quantity ratio of each light source whose color discriminability is optimum for each color can be set in advance. Thus, the observation apparatus 1 can determine the light quantity ratio of each light source on the basis of the color of the observation image and can control the plurality of light sources so that the determined light quantity ratio may be set.

According to the observation apparatus 1 to which the technology according to the present disclosure is applied, it is possible to improve the color discriminability of the observation image by automatically controlling the light quantity ratio of each light source depending on the color of the observation target.

<2. First embodiment>

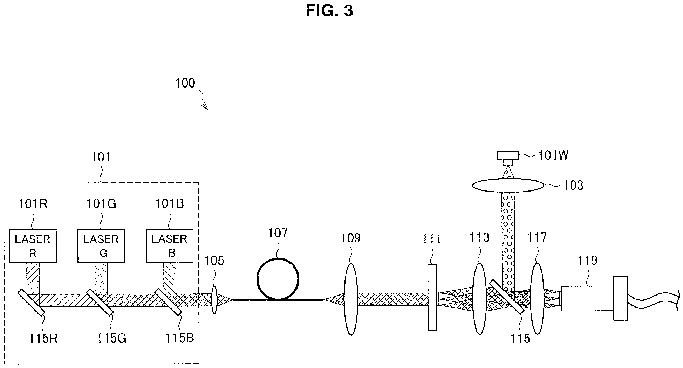

An observation apparatus according to a first embodiment of the present disclosure is now described with reference to FIGS. 3 to 6.

(2.1. Configuration of optical system of light source)

An optical system of a light source unit included in the observation apparatus according to the present embodiment is first described with reference to FIG. 3. FIG. 3 is a schematic diagram illustrated to describe the optical system of the light source unit included in the observation apparatus according to the present embodiment.

As illustrated in FIG. 3, the optical system 100 of the light source unit 10 includes a first light source 101W, a first collimating optical system 103, a second light source 101 that emits light having a wavelength spectrum different from that of the first light source 101W, an optical coupling system 105, an optical fiber 107, a third collimating optical system 109, a diffusion member 111, a second collimating optical system 113, a dichroic mirror 115, and a condenser optical system 117. In addition, although not illustrated, the first light source 101W and the second light source 101 are each provided with a control unit that controls a light emission output of each of the light sources.

The light emitted from the first light source 101W passes through the first collimating optical system 10 to produce substantially collimated light, and then enters the dichroic mirror 115. On the other hand, the light emitted from the second light source 101 sequentially passes through the optical coupling system 105, the optical fiber 107, the third collimating optical system 109, the diffusion member 111, and the second collimating optical system 113 to produce substantially collimated light, and then enters the dichroic mirror 115. The dichroic mirror 115 combines the light emitted from the first light source 101W and the light emitted from the second light source 101. The combined light is set as the observation light and enters the end portion of a light guide 119 of the lens barrel 121 via the condenser optical system 117.

The second light source 101 emits light having a wavelength spectrum different from that of the first light source 101W. Specifically, the second light source 101 includes at least one or more laser light sources that emit light of a predetermined wavelength band. In one example, the second light source 101 can include a red laser light source 101R that emits laser light in the red band (e.g., laser light having a center wavelength of about 638 nm), a green laser light source 101G that emits laser light in the green band (e.g., laser light having a center wavelength of about 532 nm), and a blue laser light source 101B that emits laser light in the blue band (e.g., laser light having a center wavelength of about 450 nm). In addition, each of the red laser light source 101R, the green laser light source 101G, and the blue laser light source 101B is provided with a collimating optical system, and each laser beam is emitted as a collimated beam of light.

Moreover, the red laser light source 101R, the green laser light source 101G, and the blue laser light source 101B can include various known laser light sources such as semiconductor laser or solid-state laser. In addition, the center wavelength of each of the red laser light source 101R, the green laser light source 101G, and the blue laser light source 101B can be controlled by the combination with a wavelength conversion mechanism.

The second light source 101 including the red laser light source 101R, the green laser light source 101G, and the blue laser light source 101B that emit light in the respective wavelength bands corresponding to three primary colors of light is capable of combining laser light emitted from each of the laser light sources, thereby generating white light. The second light source 101 is also capable of adjusting the color temperature of the combined white light by appropriately adjusting the light quantity ratio of the red laser light source 101R, the green laser light source 101G, and the blue laser light source 101B.

In the light source unit 10 of the observation apparatus 1 according to the present embodiment, however, the types of light sources of the first light source 101W and the second light source 101 are not limited to the above examples. The types of light sources of the first light source 101W and the second light source 101 are possible to be selected appropriately depending on the observation purpose, the type of the observation target 14, or the like, as long as the wavelength spectra of the emitted light are different from each other.

Further, the second light source 101 further includes dichroic mirrors 115R, 115G, and 115B that respectively reflect the laser light beams emitted from the red laser light source 101R, the green laser light source 101G, and the blue laser light source 101B. The dichroic mirrors 115R, 115G, and 115B combine the laser light beams emitted from the red laser light source 101R, the green laser light source 101G, and the blue laser light source 101B as a collimated beam of light, and emit it to the optical coupling system 105 in the subsequent stage.

Moreover, the dichroic mirrors 115R, 115G, and 115B are examples of a combining member that combines the respective laser light beams, but any other combining members can be used. In one example, as a combining member, a dichroic prism that combines light by wavelengths can be used, a polarizing beam splitter that combines light by polarization can be used, or a beam splitter that combines light by amplitude can be used.

The optical coupling system 105 includes, in one example, a condenser lens (what is called collector lens), and optically couples light emitted from the second light source 101 to the incident end of the optical fiber 107.

The optical fiber 107 guides the light emitted from the second light source 101 to the third collimating optical system 109 provided in the subsequent stage. The light emitted from the optical fiber 107 becomes a rotationally symmetric beam light, so the guidance of the light emitted from the second light source 101 by the optical fiber 107 makes it possible to make the luminance distribution in the plane of the light emitted from the second light source 101 more uniform.

Moreover, the type of the optical fiber 107 is not limited to a particular one, and it is possible to use a known multimode optical fiber (e.g., a step index multimode fiber, etc.). In addition, the core diameter of the optical fiber 107 is not limited to a particular value, and in one example, the core diameter of the optical fiber 107 can be about 1 mm.

The third collimating optical system 109 is provided in the stage following the emitting end of the optical fiber 107, and converts the light emitted from the optical fiber 107 into a collimated beam of light. The third collimating optical system 109 is capable of converting the light incident on the diffusion member 111 provided in the subsequent stage into a collimated beam of light, so it is possible to facilitate control of the light diffusion state for the diffusion member 111.

The diffusion member 111 is provided in a range near the focal position of the third collimating optical system 109 (e.g., the range of about 10% of the focal length in the front-to-back direction from the focal position), and diffuses the light emitted from the third collimating optical system 109. This allows the light emitting end of the diffusion member 111 to be regarded as a secondary light source. The light emitted from the optical fiber 107 generally produces variation in divergence angles for each combined light, so the divergence angles of the combined light are preferably unified by passing the light through the diffusion member 111.

It is possible to control the size of the secondary light source generated by the diffusion member 111 using the focal length of the third collimating optical system 109. In addition, it is possible to control the numerical aperture (NA) of the light emitted from the secondary light source generated by the diffusion member 111 using the diffusion angle of the diffusion member 111. This makes it possible to control independently both the size of the focused spot and the incident NA at the time of coupling to the end portion of the light guide 119.

Moreover, the type of the diffusion member 111 is not limited to a particular one, and various known diffusion elements can be used. Examples of the diffusion member 111 include a frosted ground glass, an opal diffusing plate in which a light diffusing substance is dispersed in glass, a holographic diffusing plate, or the like. In particular, the holographic diffusing plate is allowed to set optionally a diffusion angle of the emitting light by a holographic pattern applied on a substrate, so it can be used more suitably as the diffusion member 111.

The second collimating optical system 113 converts the light from the diffusion member 111 (i.e., the light from the secondary light source) into a collimated beam of light, and makes it incident on the dichroic mirror 115. Moreover, the light that passes through the second collimating optical system 113 is not necessarily a completely collimated beam of light, but can be divergent light of a state close to a collimated beam of light.

The first light source 101W includes, in one example, a white light source and emits white light. Although the type of the white light source including the first light source 101W is not limited to a particular one, it is selected to have a wavelength spectrum different from that of the second light source 101. In one example, the first light source 101W can be a white LED, a laser-excited phosphor, a xenon lamp, a halogen lamp, or the like. In the present embodiment, the description is given on the assumption that the first light source 101W is what is called a phosphor-based white LED using a phosphor excited by a blue LED.

The first collimating optical system 103 converts the white light emitted from the first light source 101W into a collimated beam of light, and makes the light incident on the dichroic mirror 115 in a direction different from the light passing through the second collimating optical system 113 (e.g., direction in which their optical axes are substantially orthogonal to each other). Moreover, the white light passing through the first collimating optical system 103 is not necessarily a completely collimated beam of light, which is similar to the light passing through the second collimating optical system 113.

The dichroic mirror 115 combines the light emitted from the first light source 101W and the light emitted from the second light source 101. In one example, the dichroic mirror 115 can be designed to transmit only light in a wavelength band corresponding to the light from the second light source 101 and to reflect light in other wavelength bands.

Such a dichroic mirror 115 allows the light emitted from the second light source 101 to transmit the dichroic mirror 115 and enter the condenser optical system 117. In addition, the components of the light emitted from the first light source 101W other than the wavelength band of the light emitted from the second light source 101 are reflected by the dichroic mirror 115 and enter the condenser optical system 117. This makes it possible for the dichroic mirror 115 to combine the light emitted from the first light source 101W and the light emitted from the second light source 101.

The condenser optical system 117 includes, in one example, a condenser lens, and focuses the light combined by the dichroic mirror 115 on the end portion of the light guide 119 at a predetermined paraxial lateral magnification.

In the optical system 100 described above, the image-forming magnification between the second collimating optical system 113 and the condenser optical system 117 (i.e., ratio of (focal length of the condenser optical system 117) to (focal length of the second collimating optical system 113)) is set so that the size and divergence angle of the secondary light source may match the core diameter and incident NA of the light guide. In addition, the image-forming magnification between the first collimating optical system 103 and the condenser optical system 117 (i.e., ratio of (focal length of the condenser optical system 117) to (focal length of the first collimating optical system 103)) is set so that the light from the first light source 101W matches the core diameter and incidence NA of the light guide and is coupled to the end portion of the light guide 119 with high efficiency.

The use of the light source unit 10 including such an optical system 100 makes it possible for the observation apparatus 1 to prevent the occurrence of speckle noise that occurs in using a laser light source for either the first light source 101W or the second light source 101, thereby obtaining a higher quality observation image.

(2.2. Configuration of observation apparatus)

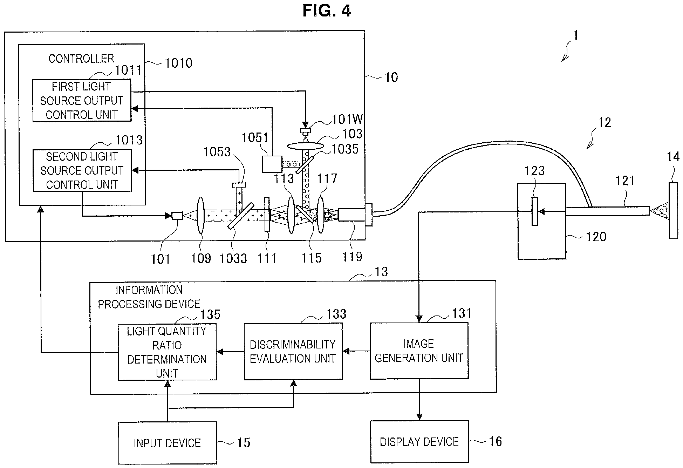

The configuration of the observation apparatus 1 according to the present embodiment is now described with reference to FIG. 4. FIG. 4 is a block diagram illustrating the configuration of the observation apparatus 1 according to the present embodiment.

As illustrated in FIG. 4, the observation apparatus 1 includes the light source unit 10, an endoscopic unit 12, the information processing device 13, the input device 15, and the display device 16.

(Light source unit)

The light source unit 10 includes a plurality of light sources that emit light beams different from each other in wavelength spectrum, and combines the light emitted from the plurality of light sources to generate observation light. The observation light generated by the light source unit 10 is guided from the end portion of the light guide 119 to the lens barrel 121 of the endoscopic unit 12 and is applied to the observation target 14 from the distal end portion of the lens barrel 121.

Here, the optical system in which the light source unit 10 generates the observation light can have a configuration similar to that of the optical system 100 described with reference to FIG. 3, or have a configuration in which a part thereof is added or omitted. Specifically, the light source unit 10 includes the first light source 101W, the first collimating optical system 103, the second light source 101 that emits light having a wavelength spectrum different from that of the first light source 101W, the third collimating optical system 109, the diffusion member 111, the second collimating optical system 113, the dichroic mirror 115, and the condenser optical system 117. These components are substantially similar in configuration and function to those of the components described with reference to FIG. 3, and so the description thereof is omitted. Moreover, in FIG. 4, the optical coupling system 105 and the optical fiber 107 are omitted for the sake of simplification of the structure of the light source unit 10.

Further, the light source unit 10 further includes a half mirror 1033, a second photodetector 1053, a half mirror 1035, a first photodetector 1051, and a controller 1010. These components are provided in the light source unit 10 to control the light emission output of the first light source 101W and the second light source 101.

The half mirror 1033 is provided, in one example, between the third collimating optical system 109 and the diffusion member 111, and splits a part of the light emitted from the second light source 101. Moreover, the split light enters the second photodetector 1053.

The second photodetector 1053 outputs the detected intensity of light to the second light source output control unit 1013. The second photodetector 1053 allows the intensity of the light emitted from the second light source 101 to be monitored, so the second light source output control unit 1013 is capable of controlling stably the intensity of the light emitted from the second light source 101.

The half mirror 1035 is provided, in one example, between the first light source 101W and the dichroic mirror 115, and splits a part of the light emitted from the first light source 101W. Moreover, the split light enters the first photodetector 1051.

The first photodetector 1051 outputs the intensity of the detected light to the first light source output control unit 1011. The first photodetector 1051 allows the intensity of the light emitted from the first light source 101W to be monitored, so the first light source output control unit 1011 is capable of controlling stably the light emitted from the first light source 101W.

Moreover, the half mirrors 1033 and 1035 are an example of a split member, but other split members can be used. In addition, the first photodetector 1051 and the second photodetector 1053 can include a known photodetector such as a photodiode or a color sensor.

The controller 1010 is a control circuit that controls the light source unit 10. Specifically, the controller 1010 includes the first light source output control unit 1011 and the second light source output control unit 1013, and controls the light emission output of each of the first light source 101W and the second light source 101. The controller 1010 includes, in one example, a processor such as CPU, microprocessor unit (MPU), or digital signal processor (DSP), and such processor executes calculation processing in accordance with a predetermined program to implement various functions.

The first light source output control unit 1011 controls the light emission output of the first light source 101W. Specifically, the first light source output control unit 1011 controls the light emission output of the first light source 101W by changing the drive current of the first light source 101W (e.g., a white LED light source). In one example, the first light source output control unit 1011 can control the output of the first light source 101W so that the intensity of the light detected by the first photodetector 1051 may be constant.

The second light source output control unit 1013 controls the light emission output of the second light source 101. Specifically, the second light source output control unit 1013 controls the light emission output of the second light source 101 by changing the drive current of the second light source 101 (e.g., a plurality of laser light sources corresponding to the respective colors of RGB). In one example, the second light source output control unit 1013 can control the output of the second light source 101 so that the intensity of the light detected by the second photodetector 1053 may be constant.

Further, in the case where the second light source 101 includes a laser light source, the second light source output control unit 1013 further executes control for making the emission wavelength of the laser light source constant by keeping the device temperature of the laser light source constant. In one example, the second light source output control unit 1013 can make the device temperature of the laser light source constant by controlling the driving of a cooling element built in the second light source 101 on the basis of the temperature information from a temperature measuring element built in the second light source 101.

Further, the first light source output control unit 1011 and the second light source output control unit 1013 change the light quantity ratio between the first light source 101W and the second light source 101 on the basis of the output from the information processing device 13. Specifically, in the observation apparatus 1 according to the present embodiment, the information processing device 13 determines the light quantity ratio between the first light source 101W and the second light source 101 on the basis of the average of the color differences between two colors calculated from the observation image. This makes it possible for the first light source output control unit 1011 and the second light source output control unit 1013 to change the light quantity ratios of the both by controlling the light emission output of the first light source 101W and the second light source 101 on the basis of the light quantity ratio determined by the information processing device 13.

(Endoscopic unit)

The endoscopic unit 12 includes the lens barrel 121 and the imaging unit 120.

The lens barrel 121 includes therein a light guide extending to the distal end portion and guides the observation light emitted from the light source unit 10 to the observation target 14. In addition, the lens barrel 121 guides light reflected from the observation target 14 to the imaging unit 120. The lens barrel 121 can be formed in a rigid, substantially cylindrical shape or can be formed in a flexible, tubular shape.

The imaging unit 120 includes an image sensor 123 capable of acquiring a color image, and photoelectrically converts light from the observation target 14 into an electric signal by the image sensor 123. Moreover, the electric signal photoelectrically converted by the image sensor 123 is output to the information processing device 13. The image sensor 123 can be various known image sensors such as a CCD image sensor and a CMOS image sensor.

(Information processing device)

The information processing device 13 generates a captured image (observation image) of the observation target 14 on the basis of the electric signal photoelectrically converted by the imaging unit 120. In addition, the information processing device 13 determines the light quantity ratio of each light source at which an average of the color differences between two colors calculated from the observation image is maximized, and outputs it to the controller 1010 of the light source unit 10. Specifically, the information processing device 13 includes an image generation unit 131, a discriminability evaluation unit 133, and a light quantity ratio determination unit 135. Moreover, the information processing device 13 can be a personal computer or the like equipped with a CPU, a ROM, a RAM, and the like.

The image generation unit 131 generates an observation image of the observation target 14 on the basis of the electric signal from the image sensor 123. The observation image generated by the image generation unit 131 is output to, in one example, the display device 16 to be visually recognized by the user. In addition, the observation image generated by the image generation unit 131 is output to, in one example, the discriminability evaluation unit 133 to be used for evaluation of color discriminability.

The discriminability evaluation unit 133 calculates a color difference between two colors from the observation image generated by the image generation unit 131. Specifically, for each pixel of the observation image, the discriminability evaluation unit 133 calculates the color difference between two colors between each pixel and four adjacent pixels, and further calculates an average of the calculated color difference between two colors for each pixel. The discriminability evaluation unit 133 can calculate the average of the color difference between two colors in pixels of the entire observation image.

The color difference between two colors is a representation expressing a difference between two colors as the distance in the L*a*b* space that is the human perceptual uniform space, and is a numerical value quantitatively expressing the difference in color tint of pixels. Thus, the calculation of the color difference between two colors between each pixel of the observation image and pixels adjacent to a noticed pixel and the calculation of the average of color differences between two colors in pixels of the entire observation image make it possible to evaluate quantitatively the degree of color discriminability in the observation image.

Further, in a case where the user is paying attention to a partial area of the observation image and the partial area is set as a noticed area, the discriminability evaluation unit 133 can calculate the average of color differences between two colors in pixels included in the set noticed area instead of the entire observation image.

In one example, in a case where biological tissues of different colors coexist in the observation image, the average of color differences between two colors in pixels of the entire observation image does not necessarily coincide with the average of color differences between two colors in pixels included in the noticed area. Thus, in a case where the noticed area to which the user is paying attention is perceptible, the discriminability evaluation unit 133 can calculate the average of color differences between two colors in pixels included in the noticed area so that the light quantity ratio of each of the light sources is determined on the basis of the color discriminability of the noticed area by the light quantity ratio determination unit 135 in the subsequent stage.

Furthermore, in a case where the user is paying attention to the difference between two points in the observation image and these two points are set as noticed points, the discriminability evaluation unit 133 can calculate the color difference between two colors in pixels of the two specified points.

In one example, in the case where there is a point where it is particularly desirable to clearly distinguish colors in the observation image for the purpose of medical examination or the like of the observation target 14, the color discriminability between pixels of two points noticed by the user can be sometimes more important than the color discriminability in the entire observation image. In such a case, the discriminability evaluation unit 133 can calculate the color difference between two colors in pixels of two points noticed by the user, so that the light quantity ratio of each of the light sources is determined on the basis of the color discriminability of the two points by the light quantity ratio determination unit 135 in the subsequent stage.

Moreover, the color difference between two colors from the captured image is calculated by, in one example, the following method. Specifically, first, RGB pixel values (i.e., values of RGB light received by the image sensor 123) of pixels in the observation image that is expressed in the sRGB (D65) color space are converted into a coordination representation in the L*a*b* color space in which the color diversity on human perception corresponds to the distance on the color space.

More specifically, first, the RGB pixel values of the observation image are converted from the sRGB values (r', g', b') to the linear RGB values (r, g, b) using the following Formula 1. Moreover, the relationships between g and g' and between b and b' are the same as the relationship between r and r' shown in Formula 1.

[Math. 1]

$$r = \begin{cases} \dfrac{r'}{12.92} & (r \le 0.040450) \\ \left(\dfrac{r' + 0.055}{1.055}\right)^{2.4} & (r > 0.040450) \end{cases} \quad \cdots \text{Formula 1}$$

Then, the converted linear RGB values (r, g, b) are converted into coordinate values (X, Y, Z) in the XYZ (D50) color space using the following Formula 2.

[Math. 2]

$$\begin{pmatrix} X \\ Y \\ Z \end{pmatrix} = \begin{pmatrix} 0.436014 & 0.385099 & 0.143161 \\ 0.222416 & 0.716916 & 0.0605853 \\ 0.0139105 & 0.0970884 & 0.714293 \end{pmatrix} \begin{pmatrix} r \\ g \\ b \end{pmatrix} \quad \cdots \text{Formula 2}$$

Subsequently, the coordinate values (X, Y, Z) in the XYZ (D50) color space are converted into coordinate values (L*, a*, b*) in the L*a*b* color space using Formulas 4 to 6 expressed as f(t) indicated in the following Formula 3.

[Math. 3]

$$f(t) = \begin{cases} t^{1/3} & \left(t > \left(\dfrac{6}{29}\right)^3\right) \\ \dfrac{\left(\left(\dfrac{29}{3}\right)^3 t + 16\right)}{116} & \left(t \le \left(\dfrac{6}{29}\right)^3\right) \end{cases} \quad \cdots \text{Formula 3}$$

$$L^* = 116 f\left(\frac{Y}{1.000}\right) - 16 \quad \cdots \text{Formula 4}$$

$$a^* = 500 \left(f\left(\frac{X}{0.9643}\right) - f\left(\frac{Y}{1.000}\right)\right) \quad \cdots \text{Formula 5}$$

$$b^* = 200 \left(f\left(\frac{Y}{1.000}\right) - f\left(\frac{Z}{0.8253}\right)\right) \quad \cdots \text{Formula 6}$$

After the conversion of the RGB pixel values of pixels in the observation image into the coordinate representation in the L*a*b* color space, the Euclidean distance in the L*a*b* color space between the relevant pixel and pixels adjacent to the relevant pixel is calculate on the basis of Formula 7. The calculated Euclidean distance is the color difference between two colors ΔE.

[Math. 4]

$$\Delta E = \sqrt{(\Delta L^*)^2 + (\Delta a^*)^2 + (\Delta b^*)^2} \quad \cdots \text{Formula 7}$$

The light quantity ratio determination unit 135 determines the light quantity ratio of each of the plurality of light sources included in the light source unit 10 on the basis of the color difference between two colors calculated by the discriminability evaluation unit 133. Specifically, the light quantity ratio determination unit 135 applies a plurality of light quantity ratio conditions to the light source unit 10, and then calculates the color difference between two colors from the observation image to which each light quantity ratio condition is applied and compares the calculated color differences between two colors to each other. Subsequently, the light quantity ratio determination unit 135 determines, as the final light quantity ratio condition, a light quantity ratio condition in which the color difference between two colors is maximized among the applied light quantity ratio conditions. The determined light quantity ratio condition is output to the controller 1010 of the light source unit 10, and the controller 1010 controls the light emission output of the first light source 101W and the second light source 101 so that the light quantity ratio determined by the light quantity ratio determination unit 135 may be set.

Moreover, the light quantity ratio determination unit 135 can determine the light quantity ratio at which the color difference between two colors calculated by the discriminability evaluation unit 133 is maximized in a processing procedure different from the above procedure. In one example, the light quantity ratio determination unit 135 gradually changes the light quantity ratio of each light source included in the light source unit 10, and can determine a light quantity ratio when the color difference between two colors calculated from the observation image has the local maximum value as the final light quantity ratio.

Further, in the case where the light quantity ratio determination unit 135 changes the light quantity ratio of each light source of the light source unit 10, the light quantity ratio determination unit 135 can determine the light quantity ratio so that the color temperature of the observation light emitted from the light source unit 10 may be constant. Specifically, the light quantity ratio determination unit 135 can allow the light quantity ratio between the plurality of light sources emitting light corresponding to each color such as red, green, and blue to be constant and can change the light quantity ratio between the plurality of light sources that emit white light. In one example, the light quantity ratio determination unit 135 can change the light quantity ratio between the first light source 101W that emits white light and the second light source 101, and can allow the light quantity ratio between the red laser light source 101R, the green laser light source 101G, and the blue laser light source 101B, which are included in the second light source 101, to be constant. This makes it possible for the color tone of the entire observation image to be significantly changed in the case where the light quantity ratio is changed by the light quantity ratio determination unit 135, thereby preventing the user from feeling uncomfortable.

(Display device)

The display device 16 displays the observation image generated by the image generation unit 131 of the information processing device 13. The display device 16 can be, in one example, a CRT display device, a liquid crystal display device, a plasma display device, an organic EL display device, or the like.

(Input device)

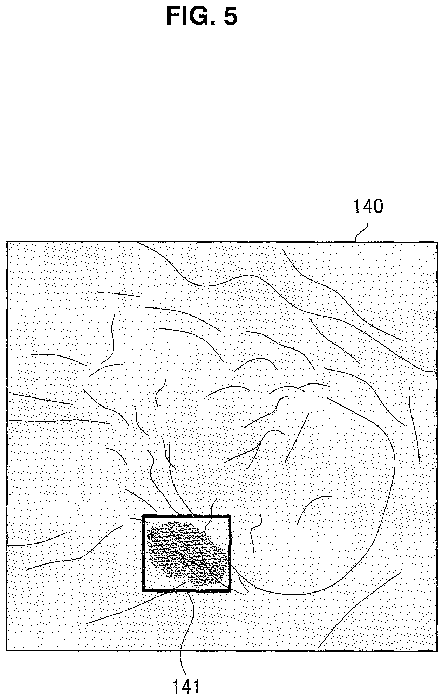

The input device 15 is an input interface for receiving an input operation by a user. Specifically, the user is able to set a noticed area or a noticed point in the observation image through the input device 15. In one example, FIG. 5 is an example of an observation image in which a noticed area is set through the input device 15.

As illustrated in FIG. 5, in one example, the user is able to set a noticed area 141 in an observation target 140 photographed in the observation image obtained by capturing the inside of the body cavity of the patient. This makes it possible for the discriminability evaluation unit 133 to calculate an average of color differences between two colors of pixels included in the noticed area 141, and makes it possible for the light quantity ratio determination unit 135 to determine a light quantity ratio so that the color discriminability of pixels included in the noticed area 141 may increase on the basis of the calculated average of the color differences between two colors. Thus, the user is able to visually recognize the observation image in which the color discriminability of the noticed area 141 is further improved.

Moreover, the user can specify optionally the light quantity ratios of the first light source 101W and the second light source 101 included in the light source unit 10 through the input device 15, and can specify a light quantity ratio selected from preset light quantity ratios. The light quantity ratio specified by the user through the input device 15 is input to the controller 1010 of the light source unit 10, and the first light source output control unit 1011 and the second light source output control unit 1013 control the first light source 101W and the second light source 101, respectively, so that the specified light quantity ratio may be achieved.

The observation apparatus 1 according to the present embodiment having the configuration described above is capable of searching and determining a light quantity ratio at which the color discriminability of the observation target 14 is satisfactory on the basis of the color difference between two colors calculated from the observation image by the discriminability evaluation unit 133. Thus, the observation apparatus 1 according to the present embodiment makes it possible to acquire an observation image having appropriate color discriminability regardless of color of the observation target 14.

(2.3. Method of controlling observation apparatus)

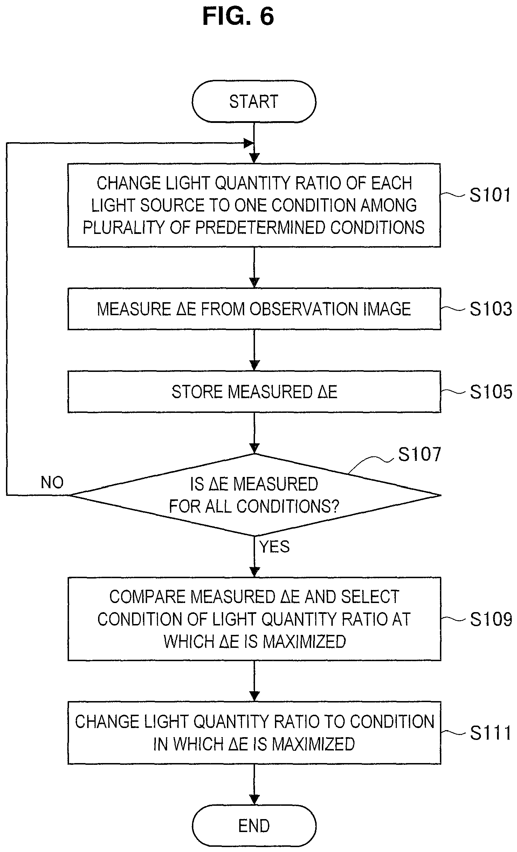

Subsequently, a method of controlling the observation apparatus 1 according to the present embodiment is described with reference to FIG. 6. FIG. 6 is a flowchart illustrated to describe an example of a method of controlling the observation apparatus 1 according to the present embodiment.

The light beams having wavelength spectra different from each other are first emitted from the first light source 101W and the second light source 101 included in the light source unit 10, and they are combined by the optical system 100 of the light source unit 10 to generate the observation light. The generated observation light is applied to the observation target 14, is reflected from the observation target 14, and then is photoelectrically converted into an electric signal by the imaging unit 120. The photoelectrically converted electric signal is input to the information processing device 13, and the information processing device 13 generates an observation image on the basis of the input electric signal.

Here, as illustrated in FIG. 6, the light quantity ratio determination unit 135 first sets the light quantity ratio of each of the light sources (the first light source 101W and the second light source 101) included in the light source unit 10 to one condition among a plurality of predetermined conditions (S101). Next, the discriminability evaluation unit 133 calculates the color difference between two colors ΔE from the observation image obtained by capturing the observation target 14 irradiated with the observation light of the light quantity ratio that is set (S103), and temporarily store the calculated color difference between two colors ΔE (S105).

Subsequently, the light quantity ratio determination unit 135 decides whether or not the color difference between two colors ΔE of the observation image is calculated for all of the plurality of predetermined light quantity ratio conditions (S107). In a case where the color difference between two colors ΔE is not calculated for all of the plurality of predetermined light quantity ratio conditions (No in S107), the light quantity ratio determination unit 135 returns the processing to S101, sets the light quantity ratio of each light source included in the light source unit 10 to another condition among a plurality of predetermined conditions, and the discriminability evaluation unit 133 again calculates the color difference between two colors.

On the other hand, in a case where the color difference between two colors ΔE is calculated for all of the plurality of predetermined light quantity ratio conditions (Yes in S107), the light quantity ratio determination unit 135 compares the color differences between two colors ΔE at the respective light quantity ratios, and selects a light quantity ratio at which the color difference between two colors ΔE is maximized as the final light quantity ratio (S109). Furthermore, the light quantity ratio determination unit 135 outputs the selected light quantity ratio to the controller 1010 of the light source unit 10, thereby changing the light quantity ratio of each light source of the light source unit 10 (S111).

Moreover, the method of controlling the observation apparatus 1 described above is merely an example, and the method of controlling the observation apparatus 1 according to the present embodiment is not limited to the above example. The observation apparatus 1 according to the present embodiment can determine the light quantity ratio at which the color difference between two colors ΔE is maximized in a procedure different from the above procedure.

<3. Second embodiment>

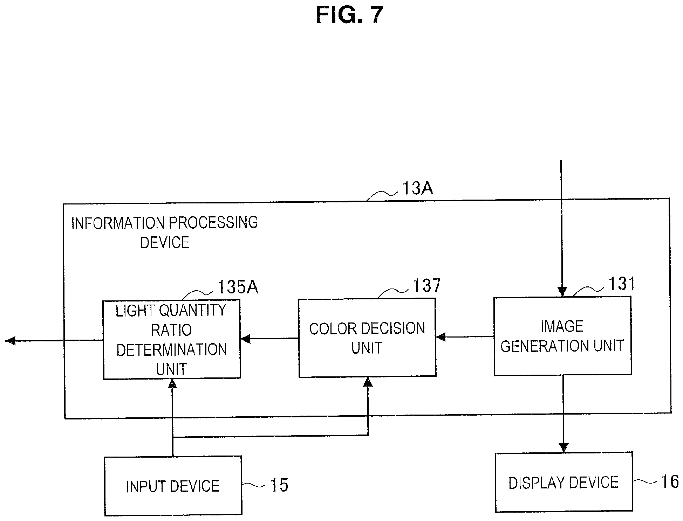

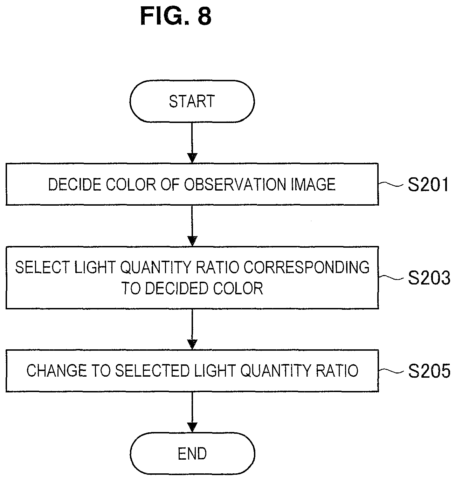

Subsequently, an observation apparatus according to a second embodiment of the present disclosure is described with reference to FIGS. 7 and 8. The observation apparatus according to the second embodiment of the present disclosure is different from the observation apparatus 1 according to the first embodiment only in an information processing device 13A. Thus, FIG. 7 illustrates only the information processing device 13A.

(3.1. Configuration of observation apparatus)

The configuration of the information processing device 13A included in the observation apparatus according to the present embodiment is now described with reference to FIG. 7. FIG. 7 is a block diagram illustrating the configuration of the information processing device 13A included in the observation apparatus according to the present embodiment. Moreover, the light source unit 10, the endoscopic unit 12, the input device 15, and the display device 16 are substantially similar in configuration and function to those described with reference to FIGS. 3 and 4, so the description thereof is omitted here.

The information processing device 13A generates a captured image (observation image) of the observation target 14 on the basis of the electric signal photoelectrically converted by the imaging unit 120, then determines the light quantity ratio of each light source on the basis of the color of the observation image and outputs it to the controller 1010 of the light source unit 10. Specifically, as illustrated in FIG. 7, the information processing device 13A includes an image generation unit 131, a color decision unit 137, and a light quantity ratio determination unit 135A. Moreover, the information processing device 13A can be a personal computer or the like equipped with a CPU, a ROM, a RAM, and the like.

The image generation unit 131 generates an observation image of the observation target 14 on the basis of the electric signal from the image sensor 123. The observation image generated by the image generation unit 131 is output to, in one example, the display device 16 to be visually recognized by the user. In addition, the observation image generated by the image generation unit 131 is output to the color decision unit 137 to be used for decision of the color of the observation image.

The color decision unit 137 decides a color of the observation image generated by the image generation unit 131. Specifically, the color decision unit 137 adds all the RGB pixel values of each pixel in the observation image and then divides it by the number of pixels, so can decide the color of the observation image from the average value of the colors of pixels in the observation image. In addition, the color decision unit 137 converts the RGB pixel values of each pixel in the observation image into coordinates in the L*a*b* color space in which the diversity of colors on human perception and the distance on the color space correspond to each other, and averages them, so can decide the color of the observation image.

As described above, the wavelength spectrum of the observation light having high color discriminability varies depending on the color of the observation target 14. Thus, the decision and setting in advance of the light quantity ratio of each light source that allows the color discriminability to be satisfactory for each color of the observation image make it possible for the information processing device 13A to determine a light quantity ratio of each light source in which the color discriminability from the color of the observation image is satisfactory.

Further, in the case where the user is paying attention to a partial area of the observation image and the partial area is set as the noticed area, the color decision unit 137 can decide the color of the observation image from the average value of the colors of pixels included in the set partial area.

In one example, in a case where a biological tissue having a color different only in a portion of the observation image is photographed, if the color of the observation image is decided from the average value of colors of pixels in the entire observation image, there is a possibility that the light quantity ratio at which the color discriminability is satisfactory is not selected for a portion having a different color. Thus, in the case where the color of the noticed area to which the user is paying attention is different from the surroundings, the color decision unit 137 calculates an average value of colors of pixels included in the noticed area, and the light quantity ratio determination unit 135A in the subsequent stage can determine the light quantity ratio of each light source on the basis of the color of the noticed area.

Furthermore, in a case where one point of the observation image to which the user is paying attention is set as the noticed point, the color decision unit 137 decides the color of the pixel at the noticed point, which is used for determination of the color of each light source by the light quantity ratio determination unit 135A in the subsequent stage.

In one example, in the case where there is a point to be particularly noticed in the observation image for the purpose such as medical examination of the observation target 14, the color of the pixel of the point noticed by the user is sometimes more important than the whole color of the observation image. In such a case, the color decision unit 137 can decide the color of the pixel of the noticed point to which the user is paying attention, and the light quantity ratio determination unit 135A in the subsequent stage can determine the light quantity ratio of each light source on the basis of the color of the noticed point.

The light quantity ratio determination unit 135A determines the light quantity ratio of each of the plurality of light sources included in the light source unit 10 on the basis of the color of the observation image decided by the color decision unit 137. Specifically, a database in which the light quantity ratio of each light source at which the color discriminability is satisfactory is determined in advance is prepared for each color of the observation image. Then, the light quantity ratio determination unit 135A can determine the light quantity ratio of each light source corresponding to the color of the observation image by referring to the database. Moreover, the determined light quantity ratio is output to the controller 1010 of the light source unit 10, and the controller 1010 controls the light emission output of the first light source 101W and the second light source 101 so that the light quantity ratio determined by the light quantity ratio determination unit 135A may be set.

In the observation apparatus according to the present embodiment having the above configuration, it is possible to determine the light quantity ratio at which the color discriminability of the observation target 14 is satisfactory on the basis of the color of the observation image decided by the color decision unit 137. This makes it possible for the observation apparatus according to the present embodiment to determine uniquely the light quantity ratio of each light source from the color of the observation image, thereby reducing the load of the calculation processing at the time of observation as compared with the first embodiment. Thus, the observation apparatus according to the present embodiment is capable of determining the light quantity ratio of each light source included in the light source unit 10 at a higher speed.

(3.2. Method of controlling observation apparatus)

Subsequently, a method of controlling the observation apparatus 1 according to the present embodiment is described with reference to FIG. 8. FIG. 8 is a flowchart illustrated to describe an example of a method of controlling the observation apparatus 1 according to the present embodiment.

The light beams having wavelength spectra different from each other are first emitted from the first light source 101W and the second light source 101 included in the light source unit 10, and they are combined by the optical system 100 of the light source unit 10 to generate the observation light. The generated observation light is applied to the observation target 14, is reflected from the observation target 14, and then is photoelectrically converted into an electric signal by the imaging unit 120. The photoelectrically converted electric signal is input to the information processing device 13A, and the information processing device 13A generates an observation image on the basis of the input electric signal.

As illustrated in FIG. 8, first, the color decision unit 137 decides the color of the observation image from the observation image obtained by capturing the observation target 14 (S201). Next, the light quantity ratio determination unit 135A selects the light quantity ratio of each light source corresponding to the color decided by the color decision unit 137 at which color discriminability is satisfactory by referring to a database or the like (S203). Furthermore, the light quantity ratio determination unit 135A outputs the selected light quantity ratio to the controller 1010 of the light source unit 10, and changes the light quantity ratio of each light source of the light source unit 10 (S205).

Moreover, the method of controlling the observation apparatus described above is merely an example, and the method of controlling the observation apparatus according to the present embodiment is not limited to the above example. The observation apparatus according to the present embodiment can determine the light quantity ratio of each light source, which corresponds to the color of the observation image, using a method different from the above method.

<4. Third embodiment>

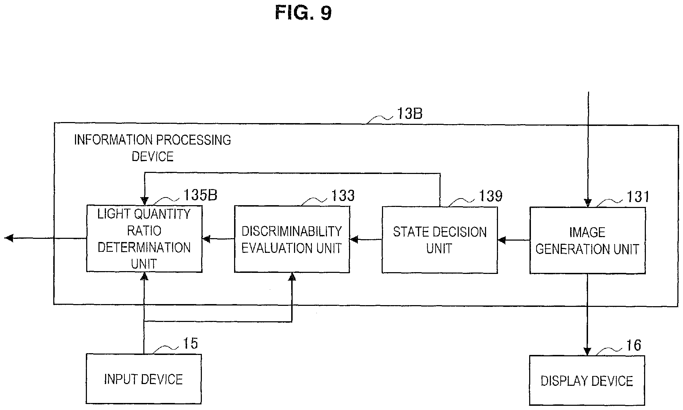

Subsequently, an observation apparatus according to a third embodiment of the present disclosure is described with reference to FIGS. 9 to 11. The observation apparatus according to the third embodiment of the present disclosure is different from the observation apparatus according to the first embodiment only in an information processing device 13B. Thus, FIG. 9 illustrates only the information processing device 13B.

(4.1. Configuration of observation apparatus)

The configuration of the information processing device 13B included in the observation apparatus according to the present embodiment is now described with reference to FIG. 9. FIG. 9 is a block diagram illustrating the configuration of the information processing device 13B included in the observation apparatus according to the present embodiment. Moreover, the light source unit 10, the endoscopic unit 12, the input device 15, and the display device 16 are substantially similar in configuration and function to those described with reference to FIGS. 3 and 4, so the description thereof is omitted here.

The information processing device 13B generates a captured image (observation image) of the observation target 14 on the basis of the electric signal photoelectrically converted by the imaging unit 120, determines a light quantity ratio of each light source appropriate for preferred one of color rendering or discriminability in the observation image, and outputs it to the controller 1010 of the light source unit 10. Specifically, as illustrated in FIG. 9, the information processing device 13B includes an image generation unit 131, a state decision unit 139, a discriminability evaluation unit 133, and a light quantity ratio determination unit 135B. Moreover, the information processing device 13B can be a personal computer or the like equipped with a CPU, a ROM, a RAM, and the like.

The image generation unit 131 generates an observation image of the observation target 14 on the basis of the electric signal from the image sensor 123. The observation image generated by the image generation unit 131 is output to, in one example, the display device 16 to be visually recognized by the user. In addition, the observation image generated by the image generation unit 131 is output to, in one example, the discriminability evaluation unit 133 to be used for evaluation of color discriminability.

The state decision unit 139 decides whether or not the state of the observation apparatus is in a color rendering priority state. Specifically, the state decision unit 139 decides whether the observation apparatus is in a state of being irradiated with observation light having high color rendering or in a state of being irradiated with observation light having high color discriminability.

This is because, in the observation apparatus, an observation image having high color discriminability for each biological tissue is sometimes necessary, and in some cases, an observation image that looks more natural like observing the observation target 14 under illumination of natural light is necessary. In one example, in a case where the entire observation target 14 is viewed from a bird's eye view, the observation apparatus can irradiate the observation target 14 with light having high color rendering closer to natural light (i.e., sunlight) and can capture the observation image that looks more natural. In addition, in a case where a particular area of the observation target 14 is observed while noticing it, the observation apparatus can irradiate the observation target 14 with light having higher color discriminability and capture an observation image having higher color discriminability, thereby improving discriminability of the tissue.

Moreover, the light having high color rendering indicates light close to natural light (i.e., sunlight) and indicates light having a high general color rendering index Ra. The general color rendering index Ra can be measured, in one example, using a method and a specification conforming to the standards defined by the International Commission on Illumination (CIE) or Japanese Industrial Standards (JIS). The observation apparatus according to the present embodiment can use, in one example, light having a high ratio of light quantity of white light emitted from the first light source 101W as light having high color rendering. However, the general color rendering index Ra of the observation light depends on the spectrum of the light emitted from each light source, so the light in which the ratio of light quantity of the white light is maximized can fail to be light whose color rendering is maximized in some cases.

Here, the state of the observation apparatus can be set to either the color rendering priority state or the color discriminability priority state by the user's input, and the state decision unit 139 can decide the state of the observation apparatus on the basis of the setting by the user's input.

Further, the state decision unit 139 can decide whether the state of the observation apparatus is the color rendering priority state or the color discriminability priority state on the basis of the distance between the endoscopic unit 12 and the observation target 14. In one example, in a case where the distance between the endoscopic unit 12 and the observation target 14 is equal to or greater than a threshold value, the state decision unit 139 can decide that the state of the observation apparatus is the color rendering priority state. In a case where the distance between the unit 12 and the observation target 14 is less than the threshold value, the state decision unit 139 can decide that the state of the observation apparatus is the color discriminability priority state. Moreover, the distance between the endoscopic unit 12 and the observation target 14 can be estimated, in one example, from the lens position when the endoscopic unit 12 focuses on the observation target 14. In addition, the distance between the endoscopic unit 12 and the observation target 14 can be estimated from the exposure time of the capturing by the endoscopic unit 12 and the total luminance of the observation image in the case where the light quantity of the observation light is kept constant.

The discriminability evaluation unit 133 calculates a color difference between two colors from the observation image generated by the image generation unit 131. Specifically, for each pixel of the observation image, the discriminability evaluation unit 133 calculates the color difference between two colors between each pixel and four adjacent pixels, and further calculates an average of the calculated color difference between two colors for each pixel. The discriminability evaluation unit 133 can calculate the average of the color difference between two colors in pixels of the entire observation image.

Further, in a case where the user is paying attention to a partial area of the observation image and the partial area is set as a noticed area, the discriminability evaluation unit 133 can calculate the average of color differences between two colors in pixels included in the set noticed area instead of the entire observation image. Furthermore, in a case where the user is paying attention to the difference between two points in the observation image and these two points are set as noticed points, the discriminability evaluation unit 133 can calculate the color difference between two colors in pixels of the two specified points.

Moreover, the details of the discriminability evaluation unit 133 are substantially similar to the configuration described in the first embodiment, so the description thereof is omitted here.

The light quantity ratio determination unit 135B determines the light quantity ratio of each of the plurality of light sources included in the light source unit 10 so that either one of color rendering or color discriminability may be high on the basis of the decision by the state decision unit 139.

Specifically, in a case where the color rendering of the light emitted from the light source unit 10 increases, the light quantity ratio determination unit 135B determines the light quantity ratio of each of the plurality of light sources from among the plurality of light sources included in the light source unit 10 so that the ratio of light quantity of the first light source 101W that emits white light may increase. In one example, the light quantity ratio determination unit 135B can determine the ratio of light quantity of each of the plurality of light sources so that the light quantity ratio of the first light source 101W that emits white light among the plurality of light sources included in the light source unit 10 may be maximized, thereby maximizing the color rendering of the light emitting from the light source unit 10. In addition, in a case where the color discriminability of the light emitted from the light source unit 10 increases, the light quantity ratio determination unit 135B determines the light quantity ratio of each of the plurality of light sources on the basis of the color difference between two colors calculated by the discriminability evaluation unit 133. Moreover, the processing procedure in the light quantity ratio determination unit 135B in the case where the light quantity ratio of each of the plurality of light sources is determined on the basis of the color difference between two colors is the same as that described in the first embodiment, so the description thereof is omitted here.

In the observation apparatus according to the present embodiment having the above configuration, it is possible to irradiate the observation target 14 with observation light capable of obtaining an observation image having appropriate characteristics depending on the state of the observation apparatus. Specifically, the observation apparatus according to the present embodiment is capable of selecting either the observation light having high color rendering or the observation light having high color discriminability depending on the setting by the user, the distance between the endoscopic unit 12 and the observation target 14, or the like, and is capable of irradiating the observation target 14. This makes it possible for the observation apparatus according to the present embodiment to capture the observation image desired by the user more appropriately.

(4.2. Method of controlling observation apparatus)

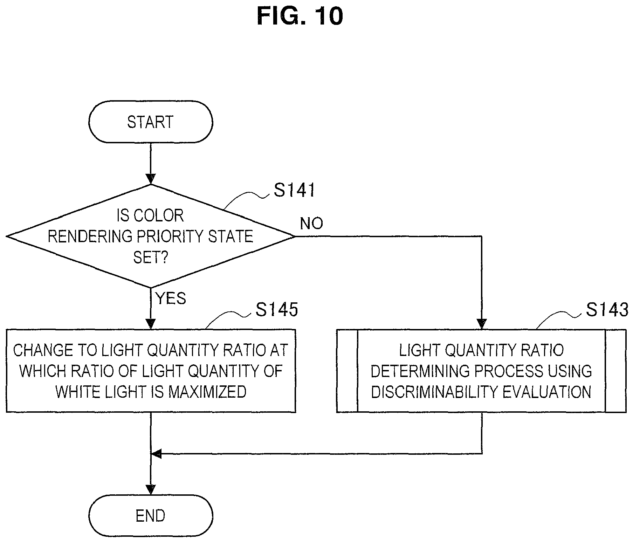

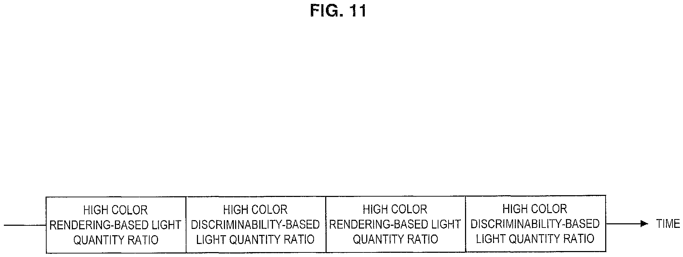

A method of controlling the observation apparatus according to the present embodiment is now described with reference to FIGS. 10 and 11. FIG. 10 is a flowchart illustrated to describe one example of a method of controlling the observation apparatus according to the present embodiment, and FIG. 11 is a diagram illustrated to describe another example of the method of controlling the observation apparatus according to the present embodiment.

An example of the method of controlling the observation apparatus according to the present embodiment is described with reference to FIG. 10. As illustrated in FIG. 10, first, the state decision unit 139 decides whether or not the observation apparatus is in the color rendering priority state (S141). Here, the setting of the observation apparatus to the color rendering priority state can be performed, in one example, by the user's input, or can be performed on the basis of the distance between the endoscopic unit 12 and the observation target 14.

In a case where the observation apparatus is not in the color rendering priority state (No in S141), the state decision unit 139 decides that the color discriminability priority state is set. Thus, the discriminability evaluation unit 133 evaluates the color discriminability of the observation image, and the light quantity ratio determination unit 135B determines the light quantity ratio on the basis of the evaluated color discriminability (S143). In a case where the light quantity ratio at which the color discriminability is high is determined, the light quantity ratio determination unit 135B outputs the determined light quantity ratio to the controller 1010 of the light source unit 10 and changes the light quantity ratio of each light source of the light source unit 10. This makes it possible for the observation apparatus to irradiate the observation target 14 with the observation light having high color discriminability. Moreover, the processing procedures of evaluating the discriminability of the observation image and determining of the light quantity ratio based on the evaluated discriminability are the same as those described in the first embodiment, so the description thereof is omitted here.

On the other hand, in a case where the observation apparatus is in the color rendering priority state (Yes in S141), the light quantity ratio determination unit 135B determines the light quantity ratio so that the ratio of light quantity of the light source emitting white light (i.e., the first light source 101W) may be maximized (S145). In a case where the ratio of light quantity of the white light is maximized and the light quantity ratio at which the color rendering of the observation light is maximized is determined, the light quantity ratio determination unit 135B outputs the determined light quantity ratio to the controller 1010 of the light source unit 10 and changes the light quantity ratio of each light source of the light source unit 10. This makes it possible for the observation apparatus to irradiate the observation target 14 with the observation light having high color rendering.

Further, another example of the method of controlling the observation apparatus according to the present embodiment is described with reference to FIG. 11. As illustrated in FIG. 11, in one example, the controller 1010 of the light source unit 10 can apply the light quantity ratio having high color rendering (high color rendering-based light quantity ratio) and the light quantity ratio having high color discriminability (high color discriminability-based light quantity ratio) to the plurality of light sources in a time division manner.

Specifically, first, the light quantity ratio determination unit 135B determines each of the light quantity ratio having high color rendering and the light quantity ratio having high color discriminability. Subsequently, the controller 1010 alternately applies the light quantity ratio having high color rendering and the light quantity ratio having high color discriminability as the light quantity ratio of the plurality of light sources. The controller 1010 can switch the light quantity ratio having high color rendering and the light quantity ratio having high color discriminability in any form. In one example, the controller 1010 can automatically switch the light quantity ratio having high color rendering and the light quantity ratio having high color discriminability every predetermined time, every one frame of a camera, or every several frames. Alternatively, the controller 1010 can switch the light quantity ratio having high color rendering and the light quantity ratio having high color discriminability on the basis of a manual operation by a user (e.g., a doctor). This makes it possible for the observation apparatus to capture individually an observation image captured with observation light having high color rendering and an observation image captured with observation light having high color discriminability. In addition, the observation apparatus is capable of causing the display device 16 to display simultaneously an observation image captured with observation light having high color rendering and an observation image captured with observation light having high color discriminability.

<5. Modification>

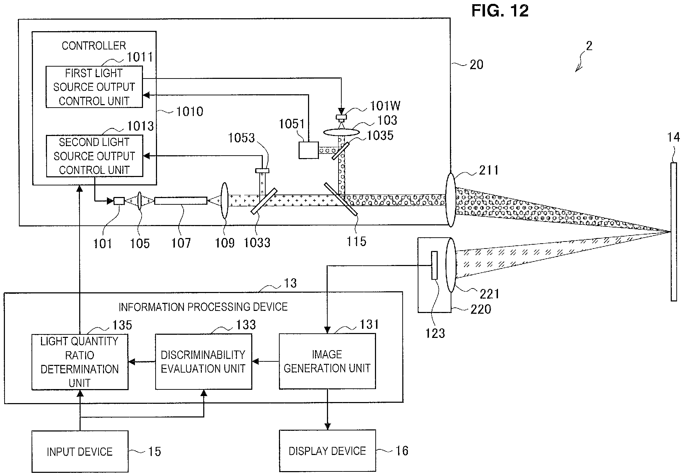

A modification of the observation apparatus according to an embodiment of the present disclosure is now described with reference to FIG. 12. The present modification is a configuration example in the case where the technology according to the present disclosure is applied to a microscopic instrument. FIG. 12 is a block diagram illustrating a configuration example in the case where the technology according to the present disclosure is applied to a microscopic instrument.

Moreover, the following description is given of an example corresponding to the observation apparatus 1 according to the first embodiment as an example.

As illustrated in FIG. 12, the observation apparatus 2 is a microscopic instrument, and includes a light source unit 20, an imaging unit 220, an information processing device 13, an input device 15, and a display device 16. Here, the information processing device 13, the input device 15, and the display device 16 are substantially similar in configuration and function to those described with reference to FIG. 4.

(Light source unit)

The light source unit 20 includes a plurality of light sources that emit light beams different from each other in wavelength spectrum, and combines the lights emitted from the plurality of light sources to generate observation light. The observation light generated by the light source unit 20 is applied onto the observation target 14 through a projection lens 211.

Here, the light source unit 20 can have a configuration similar to that of the light source unit 10 described with reference to FIG. 4, or can have a configuration in which a part thereof is added or omitted. Specifically, the light source unit 20 can include a first light source 101W, a first collimating optical system 103, a half mirror 1035, a first photodetector 1051, a second light source 101 having a wavelength spectrum different from that of the first light source 101W, a optical coupling system 105, an optical fiber 107, a third collimating optical system 109, a dichroic mirror 115, a half mirror 1033, a second photodetector 1053, and a controller 1010. These components are substantially similar in configuration and function to those of the components described with reference to FIG. 4, so the description thereof is omitted here. Moreover, in FIG. 12, the diffusion member 111 and the second collimating optical system 113 are omitted.

As illustrated in FIG. 12, the light emitted from the first light source 101W passes through the first collimating optical system 103 to produce substantially collimated light, and enters the dichroic mirror 115. On the other hand, the light emitted from the second light source 101 sequentially passes through the optical coupling system 105, the optical fiber 107, and the third collimating optical system 109 to produce substantially collimated light, and then enters the dichroic mirror 115. The dichroic mirror 115 combines the light emitted from the first light source 101W and the light beams emitted from the second light source 101. The combined light is projected on the observation target 14 as observation light through the projection lens 211 provided in the casing of the light source unit 20.

Further, a part of the light emitted from the first light source 101W is split by the half mirror 1035 and then enters the first photodetector 1051. This allows the first photodetector 1051 to detect the intensity of the light emitted from the first light source 101W, which makes it possible for the first light source output control unit 1011 to control stably the light emission output of the first light source 101W using feedback control. Furthermore, a part of the light emitted from the second light source 101 is split by the half mirror 1033 and enters the second photodetector 1053. This allows the second photodetector 105 to detect the intensity of the light emitted from the second light source 101, which makes it possible for the second light source output control unit 1013 to control stably the light emission output of the second light source 101 using feedback control.

(Imaging unit)

The imaging unit 220 includes an image sensor 123 and an image lens 221. The image lens 221 is provided in a casing of the imaging unit 220 and guides reflected light from the observation target 14 into the casing of the imaging unit 220. The light guided through the image lens 221 is photoelectrically converted into an electric signal by the image sensor 123. Moreover, the image sensor 123 is as described with reference to FIG. 4, so the description thereof is omitted here.

(Information processing device)

The information processing device 13 generates a captured image (observation image) of the observation target 14 on the basis of the electric signal photoelectrically converted by the imaging unit 220. Moreover, the configuration and function of the information processing device 13 are as described with reference to FIG. 4, so the description thereof is omitted here. In addition, the information processing device 13A according to the second embodiment described with reference to FIG. 7 or the information processing device 13B according to the third embodiment described with reference to FIG. 9 can also be used instead of the information processing device 13.

(Display device)

The display device 16 displays the observation image generated by the information processing device 13. Moreover, the configuration and function of the display device 16 are as described with reference to FIG. 4, so the description thereof is omitted here.

(Input device)

The input device 15 is an input interface for receiving an input operation by a user. Specifically, the user is able to set a noticed area or a noticed point in the observation image through the input device 15. Moreover, the configuration and function of the input device 15 are as described with reference to FIG. 4, so the description thereof is omitted here.

In other words, the technology according to the present disclosure can be similarly applied to the observation apparatus regardless of whether the observation apparatus is an endoscopic instrument or a microscopic instrument.

<6. Concluding remarks>

As described above, the inventors of the present disclosure have found that the difference in wavelength spectra of light emitted for each type of light source causes the type of light source whose color discriminability is satisfactory to be different depending on the color of the observation target 14. The observation apparatus according to an embodiment of the present disclosure conceived on the basis of this finding makes it possible to control the light quantity ratio of a plurality of light sources included in the light source unit 10, which emit light beams different from each other in wavelength spectrum, on the basis of information related to color of the observation image. Thus, the observation apparatus according to an embodiment of the present disclosure is capable of acquiring an observation image with improved color discriminability regardless of the color of the observation target 14.

Specifically, in the observation apparatus according to the first embodiment of the present disclosure, the determination of the light quantity ratio of each light source included in the light source unit 10 so that the color difference between two colors calculated from the observation image is maximized makes it possible to improve the color discriminability of the observation image. In addition, in the observation apparatus according to the second embodiment of the present disclosure, the determination of the light quantity ratio of each light source included in the light source unit 10 based on the color of the observation image makes it possible to improve the color discriminability of the observation image. Furthermore, in the observation apparatus according to the third embodiment of the present disclosure, the decision of which of color rendering or color discriminability to be given priority in the observation image and the change in light quantity ratio of each light source of the light source unit 10 make is possible to capture an observation image desired by the user more appropriately.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.

(1)
An observation apparatus including:

a plurality of light sources configured to emit light different in wavelength spectrum;
an optical system configured to emit observation light obtained by combining respective beams of light emitted from the plurality of light sources to an observation target;
an image generation unit configured to generate an observation image on the basis of light from the observation target;
a light quantity ratio calculation processing unit configured to determine a light quantity ratio of each of the plurality of light sources on the basis of information related to a color of the generated observation image; and
a controller configured to control the plurality of light sources on the basis of the determined light quantity ratio.

(2)
The observation apparatus according to (1),
in which the light quantity ratio calculation processing unit determines the light quantity ratio such that an average of color differences between two colors of pixels of the observation image and adjacent pixels is maximized.
(3)
The observation apparatus according to (2),
in which the average of color differences between two colors is an average of color differences between two colors in pixels of the entire observation image.
(4)

The observation apparatus according to (2),

in which the average of color differences between two colors is an average of color differences between two colors in pixels of a predetermined area of the observation image.

(5)

The observation apparatus according to (1),

in which the light quantity ratio calculation processing unit determines the light quantity ratio such that a color difference between two colors of two predetermined pixels is maximized.

(6)

The observation apparatus according to any one of (1) to (5),

in which the light quantity ratio calculation processing unit determines the light quantity ratio such that a color temperature is kept constant in a case of changing the light quantity ratio.

(7)

The observation apparatus according to any one of (1) to (6),

in which the light quantity ratio calculation processing unit determines a light quantity ratio at which an average of color differences between two colors is maximized by comparing respective color differences between two colors calculated from a plurality of observation images obtained by being irradiated with the observation light combined at different light quantity ratios.

(8)

The observation apparatus according to any one of (1) to (7),

in which the plurality of light sources includes a first light source configured to emit white light and a second light source configured to emit laser light at a plurality of predetermined wavelength bands.

(9)

The observation apparatus according to (8),

in which the light quantity ratio calculation processing unit determines a light quantity ratio between the first light source and the second light source.

(10)

The observation apparatus according to (8) or (9),

in which the first light source includes a white LED light source, and

the second light source includes at least a red laser light source, a green laser light source, and a blue laser light source.

(11)

The observation apparatus according to (1),

in which the light quantity ratio calculation processing unit determines the light quantity ratio on the basis of a color of the observation image.

(12)

The observation apparatus according to (11),

in which the light quantity ratio calculation processing unit determines the light quantity ratio on the basis of an average value of colors of a predetermined area of the observation image.

(13)

The observation apparatus according to (11),

in which the light quantity ratio calculation processing unit determines the light quantity ratio on the basis of a color of a predetermined pixel of the observation image.

(14)

The observation apparatus according to (9),

in which the light quantity ratio calculation processing unit decides whether or not a color rendering priority state is set, and

the light quantity ratio calculation processing unit, in a case where the color rendering priority state is not decided to be set by the light quantity ratio calculation processing unit, determines the light quantity ratio such that an average of color differences between two colors of pixels of the observation image and adjacent pixels is maximized.

(15)

The observation apparatus according to (14),

in which the light quantity ratio calculation processing unit, in a case where the color rendering priority state is decided to be set by the light quantity ratio calculation processing unit, determines the light quantity ratio such that a general color rendering index Ra is maximized.

(16)

The observation apparatus according to (9),

in which the light quantity ratio calculation processing unit determines a light quantity ratio at which an average of color differences between two colors of pixels of the observation image and adjacent pixels is maximized and determines a light quantity ratio at which a general color rendering index Ra is maximized, and the light quantity ratio between the first light source and the second light source is controlled in time division.

(17)

The observation apparatus according to any one of (1) to (16),

in which the observation apparatus is an endoscopic instrument further including a lens barrel configured to be inserted into a body cavity of a patient, guide light emitted from the optical system to an inside, and irradiate a surgical site in the body cavity with the emitted light.

(18)

A method of controlling an observation apparatus, the method including:

emitting light different from each other in wavelength spectrum from a plurality of light sources;
emitting observation light obtained by combining respective beams of emitted light to an observation target;
generating an observation image on the basis of light from the observation target;
determining, by a calculation processing device, a light quantity ratio of each of the plurality of light sources on the basis of information related to a color of the generated observation image; and
controlling the plurality of light sources on the basis of the determined light quantity ratio.

Reference Signs List

| | |
|---|---|
| 1, 2 | observation apparatus |
| 10, 20 | light source unit |
| 12 | endoscopic unit |
| 13, 13A, 13B | information processing device |
| 14 | observation target |
| 15 | input device |
| 16 | display device |
| 100 | optical system |
| 101W | first light source |
| 101 | second light source |
| 120 | imaging unit |
| 121 | lens barrel |
| 123 | image sensor |
| 131 | image generation unit |
| 133 | discriminability evaluation unit |
| 135, 135A, 135B | light quantity ratio determination unit |
| 137 | color decision unit |
| 139 | state decision unit |
| 1010 | controller |
| 1011 | first light source output control unit |
| 1013 | second light source output control unit |

**Claims**

1. An observation apparatus comprising:

   a plurality of light sources configured to emit light different in wavelength spectrum;
   an optical system configured to emit observation light obtained by combining respective beams of light emitted from the plurality of light sources to an observation target;
   an image generation unit configured to generate an observation image on a basis of light from the observation target;
   a light quantity ratio calculation processing unit configured to determine a light quantity ratio of each of the plurality of light sources on a basis of information related to a color of the generated observation image; and
   a controller configured to control the plurality of light sources on a basis of the determined light quantity ratio.

2. The observation apparatus according to claim 1,

wherein the light quantity ratio calculation processing unit determines the light quantity ratio such that an average of color differences between two colors of pixels of the observation image and adjacent pixels is maximized.

3. The observation apparatus according to claim 2,
wherein the average of color differences between two colors is an average of color differences between two colors in pixels of the entire observation image.

4. The observation apparatus according to claim 2,
wherein the average of color differences between two colors is an average of color differences between two colors in pixels of a predetermined area of the observation image.

5. The observation apparatus according to claim 1,
wherein the light quantity ratio calculation processing unit determines the light quantity ratio such that a color difference between two colors of two predetermined pixels is maximized.

6. The observation apparatus according to claim 1,
wherein the light quantity ratio calculation processing unit determines the light quantity ratio such that a color temperature is kept constant in a case of changing the light quantity ratio.

7. The observation apparatus according to claim 1,
wherein the light quantity ratio calculation processing unit determines a light quantity ratio at which an average of color differences between two colors is maximized by comparing respective color differences between two colors calculated from a plurality of observation images obtained by being irradiated with the observation light combined at different light quantity ratios.

8. The observation apparatus according to claim 1,
wherein the plurality of light sources includes a first light source configured to emit white light and a second light source configured to emit laser light at a plurality of predetermined wavelength bands.

9. The observation apparatus according to claim 8,
wherein the light quantity ratio calculation processing unit determines a light quantity ratio between the first light source and the second light source.

10. The observation apparatus according to claim 8,
wherein the first light source includes a white LED light source, and
the second light source includes at least a red laser light source, a green laser light source, and a blue laser light source.

11. The observation apparatus according to claim 1,
wherein the light quantity ratio calculation processing unit determines the light quantity ratio on a basis of a color of the observation image.

12. The observation apparatus according to claim 11,
wherein the light quantity ratio calculation processing unit determines the light quantity ratio on a basis of an average value of colors of a predetermined area of the observation image.

13. The observation apparatus according to claim 11,
wherein the light quantity ratio calculation processing unit determines the light quantity ratio on a basis of a color of a predetermined pixel of the observation image.

14. The observation apparatus according to claim 9,
wherein the light quantity ratio calculation processing unit decides whether or not a color rendering priority state is set, and
the light quantity ratio calculation processing unit, in a case where the color rendering priority state is not decided to be set by the light quantity ratio calculation processing unit, determines the light quantity ratio such that an average of color differences between two colors of pixels of the observation image and adjacent pixels is maximized.

15. The observation apparatus according to claim 14,

wherein the light quantity ratio calculation processing unit, in a case where the color rendering priority state is decided to be set by the light quantity ratio calculation processing unit, determines the light quantity ratio such that a general color rendering index Ra is maximized.

16. The observation apparatus according to claim 9,
wherein the light quantity ratio calculation processing unit determines a light quantity ratio at which an average of color differences between two colors of pixels of the observation image and adjacent pixels is maximized and determines a light quantity ratio at which a general color rendering index Ra is maximized, and
the light quantity ratio between the first light source and the second light source is controlled in time division.

17. The observation apparatus according to claim 1,
wherein the observation apparatus is an endoscopic instrument further including a lens barrel configured to be inserted into a body cavity of a patient, guide light emitted from the optical system to an inside, and irradiate a surgical site in the body cavity with the emitted light.

18. A method of controlling an observation apparatus, the method comprising:

emitting light different from each other in wavelength spectrum from a plurality of light sources;
emitting observation light obtained by combining respective beams of emitted light to an observation target;
generating an observation image on a basis of light from the observation target;
determining, by a calculation processing device, a light quantity ratio of each of the plurality of light sources on a basis of information related to a color of the generated observation image; and
controlling the plurality of light sources on a basis of the determined light quantity ratio.

# FIG. 1

# FIG. 2

FIG. 3

## FIG. 4

# FIG. 5

# FIG. 6

START

CHANGE LIGHT QUANTITY RATIO OF EACH LIGHT SOURCE TO ONE CONDITION AMONG PLURALITY OF PREDETERMINED CONDITIONS — S101

MEASURE $\Delta E$ FROM OBSERVATION IMAGE — S103

STORE MEASURED $\Delta E$ — S105

S107

NO ← IS $\Delta E$ MEASURED FOR ALL CONDITIONS?

YES

COMPARE MEASURED $\Delta E$ AND SELECT CONDITION OF LIGHT QUANTITY RATIO AT WHICH $\Delta E$ IS MAXIMIZED — S109

CHANGE LIGHT QUANTITY RATIO TO CONDITION IN WHICH $\Delta E$ IS MAXIMIZED — S111

END

# FIG. 7

~13A

INFORMATION PROCESSING
DEVICE

~135A

LIGHT QUANTITY
RATIO
DETERMINATION
UNIT

~137

COLOR DECISION
UNIT

~131

IMAGE
GENERATION UNIT

INPUT DEVICE  ~15

DISPLAY DEVICE  ~16

# FIG. 8

```
        ┌─────────────┐
        │    START    │
        └─────────────┘
               │
               ▼
┌─────────────────────────────────────┐
│  DECIDE COLOR OF OBSERVATION IMAGE   │──── S201
└─────────────────────────────────────┘
               │
               ▼
┌─────────────────────────────────────┐
│ SELECT LIGHT QUANTITY RATIO CORRESPONDING │──── S203
│         TO DECIDED COLOR             │
└─────────────────────────────────────┘
               │
               ▼
┌─────────────────────────────────────┐
│ CHANGE TO SELECTED LIGHT QUANTITY RATIO │──── S205
└─────────────────────────────────────┘
               │
               ▼
        ┌─────────────┐
        │     END     │
        └─────────────┘
```

FIG. 9

~13B

INFORMATION PROCESSING
DEVICE

~135B     ~133     ~139     ~131

| LIGHT QUANTITY RATIO DETERMINATION UNIT | DISCRIMINABILITY EVALUATION UNIT | STATE DECISION UNIT | IMAGE GENERATION UNIT |

~15

| INPUT DEVICE |

~16

| DISPLAY DEVICE |

# FIG. 10

```
                    ┌─────────────┐
                    │    START    │
                    └─────────────┘
                           │
                           ▼
                                              ⌒ S141
                      ◇ IS COLOR ◇
                   RENDERING PRIORITY STATE        NO
                          SET?
```

```
IS COLOR
RENDERING PRIORITY STATE
SET?                                    NO

YES
          ⌒ S145                                    ⌒ S143
┌──────────────────────────────┐      ┌──────────────────────────────┐
│ CHANGE TO LIGHT QUANTITY RATIO AT │  ║ LIGHT QUANTITY RATIO         ║
│ WHICH RATIO OF LIGHT QUANTITY OF  │  ║ DETERMINING PROCESS USING    ║
│ WHITE LIGHT IS MAXIMIZED          │  ║ DISCRIMINABILITY EVALUATION  ║
└──────────────────────────────┘      └──────────────────────────────┘

            ┌─────────────┐
            │     END     │
            └─────────────┘
```

FIG. 11

| HIGH COLOR RENDERING-BASED LIGHT QUANTITY RATIO | HIGH COLOR DISCRIMINABILITY-BASED LIGHT QUANTITY RATIO | HIGH COLOR RENDERING-BASED LIGHT QUANTITY RATIO | HIGH COLOR DISCRIMINABILITY-BASED LIGHT QUANTITY RATIO |
|---|---|---|---|

→ TIME

**FIG. 12**

CONTROLLER

1011

FIRST LIGHT SOURCE OUTPUT CONTROL UNIT

1010

1013

SECOND LIGHT SOURCE OUTPUT CONTROL UNIT

20

2

101W
103
1035

1051

14

1053

211

101  105  107  109  1033

115

221
220

123

13

INFORMATION PROCESSING DEVICE

135

LIGHT QUANTITY RATIO DETERMINATION UNIT

133

DISCRIMINABILITY EVALUATION UNIT

131

IMAGE GENERATION UNIT

INPUT DEVICE  15

DISPLAY DEVICE  16

<div align="center">

**INTERNATIONAL SEARCH REPORT**

</div>

| | International application No. |
|---|---|
| | PCT/JP2017/016461 |

**A. CLASSIFICATION OF SUBJECT MATTER**
*A61B1/06*(2006.01)i, *A61B1/045*(2006.01)i, *G02B23/26*(2006.01)i

According to International Patent Classification (IPC) or to both national classification and IPC

**B. FIELDS SEARCHED**

Minimum documentation searched (classification system followed by classification symbols)
A61B1/00-1/32, G02B23/26

Documentation searched other than minimum documentation to the extent that such documents are included in the fields searched

| Jitsuyo Shinan Koho | 1922–1996 | Jitsuyo Shinan Toroku Koho | 1996–2017 |
|---|---|---|---|
| Kokai Jitsuyo Shinan Koho | 1971–2017 | Toroku Jitsuyo Shinan Koho | 1994–2017 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 2015-11127 A (Olympus Corp.),<br>19 January 2015 (19.01.2015),<br>paragraphs [0029] to [0086]; fig. 1<br>& US 2016/0106299 A1<br>paragraphs [0048] to [0134]; fig. 1<br>& WO 2014/208417 A1 & EP 3015899 A1<br>& CN 105339828 A | 1,6,11,17,18<br>8-10,12,13<br>2-5,7,14-16 |
| X | JP 2012-115372 A (Fujifilm Corp.),<br>21 June 2012 (21.06.2012),<br>paragraphs [0041], [0056], [0077]; fig. 2<br>(Family: none) | 1,11,17,18 |
| Y | JP 2012-010981 A (Fujifilm Corp.),<br>19 January 2012 (19.01.2012),<br>paragraphs [0078] to [0083]; fig. 22, 23<br>(Family: none) | 8-10 |

| ☒ Further documents are listed in the continuation of Box C. | ☐ See patent family annex. |
|---|---|

| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
|---|---|

| Date of the actual completion of the international search<br>06 July 2017 (06.07.17) | Date of mailing of the international search report<br>18 July 2017 (18.07.17) |
|---|---|
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

| International application No. |
|---|
| PCT/JP2017/016461 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2006-087764 A  (Kyocera Corp.),<br>06 April 2006 (06.04.2006),<br>paragraphs [0045] to [0050]; fig. 1 to 3<br>(Family: none) | 8-10 |
| Y | JP 2016-064281 A  (Olympus Corp.),<br>28 April 2016 (28.04.2016),<br>paragraphs [0020], [0021]; fig. 13<br>(Family: none) | 12,13 |
| A | JP 2010-213746 A  (Fujifilm Corp.),<br>30 September 2010 (30.09.2010),<br>abstract<br>(Family: none) | 1-18 |

Form PCT/ISA/210 (continuation of second sheet) (January 2015)

**REFERENCES CITED IN THE DESCRIPTION**

**Patent documents cited in the description**

- JP 2011010998 A

- JP 2015091351 A